(19) Europäisches Patentamt
European Patent Office
Office européen des brevets

(11) Veröffentlichungsnummer: **0 122 448**
**B1**

(12) # EUROPÄISCHE PATENTSCHRIFT

(45) Veröffentlichungstag der Patentschrift: **19.02.86**

(51) Int. Cl.⁴: **C 07 C 50/24**, C 07 C 153/07, C 07 D 333/72

(21) Anmeldenummer: **84102632.1**

(22) Anmeldetag: **10.03.84**

(54) **2,3,5,6-Tetrasubstituierte p-Benzochinone, ihre Herstellung und Verwendung.**

(30) Priorität: **18.03.83 DE 3309719**

(43) Veröffentlichungstag der Anmeldung:
**24.10.84 Patentblatt 84/43**

(45) Bekanntmachung des Hinweises auf die Patenterteilung:
**19.02.86 Patentblatt 86/8**

(84) Benannte Vertragsstaaten:
**AT BE CH DE FR GB IT LI NL SE**

(56) Entgegenhaltungen:
**EP - A - 0 050 212**

**Chemical Abstracts Band 82, Nr. 15, 14. April 1975, Columbus, Ohio, USA M.P. CAVA et al. "Nonclassical condensed thiophenes. III. Benzo1,2-c:4,5-c'idithiophene system", Seite 436, Spalte 1, Abstract Nr. 97960s**
**Chemical Abstracts Band 77, Nr. 7, 14. August 1972, Columbus, Ohio, USA D.W.H. McDOWELL et al. "Thiophene analogs of anthraquinone", Seiten 474-475, Spalten 2, 1, Abstract Nr. 48296t**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Steiner, Gerd, Dr., Oberer Waldweg 1, D-6719 Kirchheim (DE)**

EP 0 122 448 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 2,3,5,6-tetrasubstituierte p-Benzochinone, deren Herstellung sowie deren Verwendung zur Herstellung von 4,8-Dihydro-benzo[1,2-c:4,5-c']-dithiophen-4,8-dion.

Die bisherige Darstellung des 4,8-Dihydrobenzo[1,2-c:4,5-c']-dithiophen-4,8-dions nach D.W.H. Mac Dowell und J.C. Wisowaty, J. Org. Chem. *37*, 1712 (1972) ist langwierig und verläuft besonders bei grösseren Ansätzen mit recht mässigen Ausbeuten.

Es wurde nun gefunden, dass man das 4,8-Dihydro-benzo[1,2-c:4,5-c']-dithiophen-4,8-dion aus neuen Verbindungen auf relativ einfache Weise erhalten kann.

Gegenstand der Erfindung sind 2,3,5,6-tetrasubstituierte p-Benzochinone der Formel I

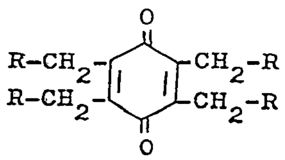

(I)

worin R ein Bromatom, einen Thio-$C_2$-$C_6$-carbonsäurerest oder einen Benzoylthiorest bedeutet.

Als Thio-$C_2$-$C_6$-carbonsäurerest der Formel $CH_3-(CH_2)_{0-4}-CO-S-$ ist vorzugsweise der Acetylthiorest $CH_3-CO-S$ zu nennen.

Gegenstand der Erfindung ist weiter ein Verfahren zur Herstellung der 2,3,5,6-tetrasubstituierten p-Benzochinone der Formel I, welches darin besteht, dass man 2,3,5,6-Tetramethylhydrochinon oder 2,3,5,6-Tetramethyl-p-benzochinon unter Licht bei erhöhter Temperatur mit Brom umsetzt und – falls gewünscht – das so erhaltene 2,3,5,6-Tetra-(brommethyl)-p-benzochinon mit einer Thiocarbonsäure der Formel R'–H unsetzt, worin R' dasselbe wie R – ausgenommen Brom – bedeutet.

Die Bromierung von 2,3,5,6-Tetramethylhydrochinon erfolgt zweckmässig in Gegenwart einer Quecksilberlampe in einem inerten Lösungsmittel, vorzugsweise einem Halogenkohlenwasserstoff, wie z.B. Tetrachlorkohlenstoff, Chloroform, 1,1,1-Trichlorethan, 1,1-Dichlorethan, Propylenchlorid, Perchlorethylen und Tetrachlorethan bei Temperaturen zwischen Raumtemperatur und 150°C, vorzugsweise bei 50-80°C. Das dabei entstehende neue 2,3,5,6-Tetra-(brommethyl)-p-benzochinon wird als kristalline Verbindung isoliert.

Anstelle von 2,3,5,6-Tetramethyl-hydrochinon kann man auch in analoger Weise 2,3,5,6-Tetramethyl-p-benzochinon bromieren.

Das 2,3,5,6-Tetra-(brommethyl)-p-benzochinon wird gewünschtenfalls mit einer Thiocarbonsäure, wie z.B. Thiobenzoesäure oder Thioessigsäure, in einem inerten organischen Lösungsmittel, wie z.B. einem Kohlenwasserstoff oder einem Ether, in Gegenwart einer Base, wie z.B. einem Alkali- oder Erdalkalicarbonat oder einem Metallhydroxid, bei Temperaturen zwischen 0 und 120°C zum 2,3,5,6-Tetra-(thiocarbonsäure-methyl)-p-benzochinon umgesetzt, welches als kristalline Substanz isoliert wird.

Gegenstand der Erfindung ist schliesslich auch die Verwendung der Verbindungen der Formel I zur Herstellung von 4,8-Dihydro-benzo[1,2-c:4,5-c']dithiophen-4,8-dion.

Diese Umsetzung erfolgt in Gegenwart einer starken Base, vorzugsweise einem Alkalihydroxid, in einem inerten organischen Lösungsmittel, vorzugsweise einem Alkohol wie Methanol, Ethanol oder einem Dialkohol, vorzugsweise Ethylenglykol, unter Einleiten von Druckluft zur Dehydrierung des primär entstehenden Tetrahydro-Derivates bei Temperaturen zwischen 50 und 150°C, vorzugsweise 70-100°C.

Man kann das 2,3,5,6-Tetra-(brommethyl)-p-benzochinon der Formel I auch direkt durch Umsetzung mit einem wasserfreien Alkalisulfid in einem inerten organischen Lösungsmittel, vorzugsweise einem Alkohol oder Dialkohol wie Ethylenglykol, bei Temperaturen zwischen 50 und 150°C und anschliessender Dehydrierung des Tetrahydro-Derivates mit Sulfurylchlorid in einem inerten Lösungsmittel, z.B. einem Halogenkohlenwasserstoff wie Methylenchlorid oder Chloroform, in das 4,8-Dihydro-benzo[1,2-c:4,5-c']dithiophen-4,8-dion überführen.

Bei der letzteren direkten Cyclisierungsreaktion zum 4,8-Dihydro-benzo[1,2-c:4,5-c']dithiophen-4,8-dion ist der Ausschluss des Sauerstoffs der Luft und das Arbeiten unter Inertgas, beispielsweise Stickstoff, von Vorteil.

Das 4,8-Dihydro-benzo[1,2-c:4,5-c']-dithiophen-4,8-dion ist ein wertvolles Ausgangsmaterial zur Herstellung der in der EP-OS 0050212 beschriebenen 5-substituierten 9-Cyanmethylen-dithieno[3,4-b:4',3'-e]-azepine. Mit dessen Hilfe lassen sich diese Azepine auf einfachere Weise kostengünstiger und mit wesentlich besserer Ausbeute, vor allem bei grösseren Ansätzen, herstellen.

Die folgenden Beispiele dienen zur Erläuterung der Erfindung.

*Beispiel 1:*

*2,3,5,6-Tetra-(brommethyl)-p-benzochinon*

40,0 g (240 mM) 2,3,5,6-Tetramethyl-hydrochinon in 1200 ml Tetrachlorkohlenstoff wurden in eine 2 l Rührapparatur, die mit einer 125 W Quecksilber-Hochdrucklampe (Philips HPK) ausgestattet war, gerührt und auf Rückflusstemperatur erhitzt. Nach dem Einschalten der Lampe tropfte man unter intensivem Rühren 204 g (1272 mM) Brom im Verlauf von 45 min unter ständigem Rückflusskochen hinzu. Man liess noch 1 h unter Bestrahlung nachrühren, und überführte anschliessend das Reaktionsgemisch in einen 2 l Erlenmeyerkolben. Beim Abkühlen kristallisierte das Produkt aus. Nach 4stündigem Kühlen im Eisbad saugte man die fast farblosen Kristalle ab. Man isolierte 91,4 g (79%) Produkt, Schmelzpunkt 169-172°C.

*Beispiel 2:*

*2,3,5,6-Tetra-(benzoylthiomethyl)-p-benzochinon*

44,0 g (329 mM) fein pulverisiertes Kaliumcarbonat wurden in 350 ml absolutem Toluol suspendiert und mit 19,2 g (40 mM) 2,3,5,6-Tetra-(brommethyl)-p-benzochinon (fein pulverisiert) versetzt. Anschliessend tropfte man 22,0 g (160 mM) Thiobenzoesäure innerhalb 5 min unter gutem Rühren hinzu. Man heizte anschliessend das Reaktionsgemisch langsam auf. Bei 60°C Innentemperatur sprang die Reaktion an (Dunkelfärbung, Temperaturerhöhung und Ausfallen von Festkörpern). Man heizte noch 10 min bei 100°C Badtemperatur nach und liess dann unter Rühren, zuletzt im Eisbad, abkühlen. Die Festkörper wurden abgesaugt, mit Toluol und anschliessend mit reichlich Wasser ausgewaschen. Man isolierte 23,0 g (81%) Produkt, Schmelzpunkt 224-226°C.

*Beispiel 3:*

*2,3,5,6-Tetra-(acetyl-thiomethyl)-p-benzochinon*

41,3 g (300 mM) fein pulverisiertes Kaliumcarbonat wurden in 350 ml absolutem Toluol unter Eiskühlung suspendiert und mit 18,0 g (37,5 mM) 2,3,5,6-Tetra-(brommethyl)-p-benzochinon (fein pulverisiert) versetzt. Anschliessend tropfte man 12,5 g (150 mM) Thioessigsäure im Verlauf von 5-7 min unter gutem Rühren hinzu. Man liess das Reaktionsgemisch noch 1 h im Eisbad nachrühren und saugte die ausgefallenen Festkörper unter Nachwaschen mit Toluol und anschliessend mit reichlich Wasser ab. Man isolierte 12,3 g (70%) Produkt, Schmelzpunkt 133-135°C.

*Verwendungsbeispiele*

*Beispiel A:*

48,5 g (68,5 mM) 2,3,5,6-Tetra-(benzoyl-thiomethyl)-p-benzochinon wurden in 450 ml Ethylenglykol suspendiert und mit 32,8 g (411 mM) 50%iger Natronlauge unter intensivem Rühren versetzt. Man leitete anschliessend Druckluft in einem kräftigen Strahl durch das Reaktionsgemisch hindurch und heizte langsam auf. Bei 70°C Innentemperatur wurde die dunkle Mischung homogen. Man steigerte die Innentemperatur auf 90-100°C und rührte bei dieser Temperatur 5 h unter ständigem Durchleiten von Druckluft intensiv nach. Nach dem Abkühlen gab man 700 ml Wasser hinzu und liess noch 0,5 h unter Eiskühlung nachrühren. Man saugte die Festkörper ab und wusch mit reichlich Wasser nach. Man isolierte 11,0 g (73%) 4,8-Dihydro-benzo[1,2-c:4,5-c']dithiophen-4,8-dion, Schmelzpunkt 340°C.

*Beispiel B:*

In eine 0,5 l Rührapparatur, die mit Aluminium-Folie vollständig abgedunkelt war, gab man 250 ml absolutes Ethylenglykol und erhitzte unter Stickstoff auf 140-150°C Innentemperatur. Uber zwei Festkörper-Dosiervorrichtungen fügte man kontinuierlich 9,6 g (20 mM) 2,3,5,6-Tetra-(brommethyl)-p-benzochinon und 3,9 g (50 mM) wasserfreies Natriumsulfid (jeweils fein pulverisiert) im Verlauf von 20 min unter intensivem Rühren hinzu. Man liess das Reaktionsgemisch noch 45 min nachrühren und saugte heiss unter Nachwaschen mit heissem Ethylenglykol ab. Das Filtrat goss man in 3 l Wasser und säuerte unter Rühren mit verdünnter Salzsäure an. Die ausgefallenen Festkörper wurden abgesaugt und mit Wasser ausgewaschen. Man isolierte 4,0 g des Tetrahydro-Derivates, das nach sorgfältigem Trocknen in 200 ml Methylenchlorid suspendiert und unter Rühren mit 2,9 ml (36 mM) Sulfurylchlorid versetzt wurde. Man liess noch 1 h bei Rückflusstemperatur nachrühren, engte die Reaktionsmischung auf die Hälfte ein, kühlte im Eisbad und saugte die ausgefallenen Festkörper ab. Weiteres Einengen der Mutterlauge ergab eine Nachfällung. Man isolierte 1,1 g (25%) 4,8-Dihydro-benzo[1,2-c:4,5-c']-dithiophen-4,8-dion, Schmelzpunkt 340°C.

**Patentansprüche**

1. 2,3,5,6-Tetrasubstituierte p-Benzochinone der Formel I

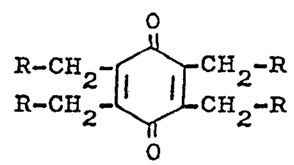

(I)

worin R ein Bromatom, einen Thio-$C_2$-$C_6$-carbonsäurerest oder einen Benzoylthiorest bedeutet.

2. 2,3,5,6-Tetra-(brommethyl)-p-benzochinon.

3. 2,3,5,6-Tetra-(benzoyl-thiomethyl)-p-benzochinon.

4. 2,3,5,6-Tetra-(acetyl-thiomethyl)-p-benzochinon.

5. Verfahren zur Herstellung der 2,3,5,6-tetrasubstituierten p-Benzochinone der Formel I gemäss Anspruch 1, dadurch gekennzeichnet, dass man 2,3,5,6-Tetramethylhydrochinon oder 2,3,5,6-Tetramethyl-p-benzochinon unter Licht bei erhöhter Temperatur mit Brom umsetzt und – falls gewünscht – das so erhaltene 2,3,5,6-Tetra-(brommethyl)-p-benzochinon mit einer Thiocarbonsäure der Formel R'–H umsetzt, worin R' dasselbe wie R – ausgenommen Brom – bedeutet.

6. Verwendung der Verbindungen der Formel I gemäss Anspruch 1 zur Herstellung von 4,8-Dihydro-benzo[1,2-c:4,5-c']-dithiophen-4,8-dion.

**Claims**

1. A 2,3,5,6-tetrasubstituted p-benzoquinone of the formula I

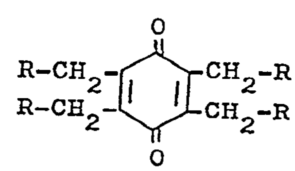

(I)

where R is bromine, $C_2$-$C_6$-acylthio or benzoylthio.

2. 2,3,5,6-Tetra-(bromomethyl)-p-benzoquinone.

3. 2,3,5,6-Tetra(benzoylthiomethyl)-p-benzoquinone.

4. 2,3,5,6-Tetra-(acetylthiomethyl)-p-benzoquinone.

5. A process for the preparation of a 2,3,5,6-tetrasubstituted p-benzoquinone of the formula I as claimed in claim 1, wherein 2,3,5,6-tetramethylhydroquinone or 2,3,5,6-tetramethyl-p-benzoquinone is reacted with bromine at elevated temperature with exposure to light, and, if desired, the resulting 2,3,5,6-tetra-(bromomethyl)-p-benzoquinone is reacted with a thiocarboxylic acid of the formula R'−H, where R' has the same meanings as R, with the exception of bromine.

6. The use of a compound of the formula I as claimed in claim 1 for the preparation of 4,8-dihydrobenzo[1,2-c:4,5-c']-dithiophene-4,8-dione.

## Revendications

1. p-Benzoquinones tétrasubstituées en 2,3,5,6 de formule I

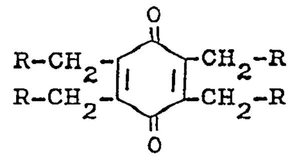

(I)

où R représente un atome de brome, un reste acide thiocarboxylique en $C_2$-$C_6$ ou un reste benzoylthio.

2. 2,3,5,6-Tétra-(bromométhyl)-p-benzoquinone.

3. 2,3,5,6-Tétra-(benzoyl-thiométhyl)-p-benzoquinone.

4. 2,3,5,6-Tétra-(acétyl-thiométhyl)-p-benzoquinone.

5. Procédé de préparation de p-benzoquinones tétrasubstituées en 2,3,5,6 de formule I selon la revendication 1, caractérisé par le fait que l'on fait réagir, à la lumière et à température élevée, de la 2,3,5,6-tétraméthylhydroquinone ou 2,3,5,6-tétraméthyl-p-benzoquinone avec du brome et – si on le désire – on fait réagir la 2,3,5,6-tétra-(bromométhyl)-p-benzoquinone obtenue avec un acide thiocarboxylique de formule R'−H, où R' représente la même chose que R – à l'exception du brome.

6. Utilisation des composés de formule I selon la revendication 1 pour la préparation de 4,8-dihydro-benzo[1,2-c:4,5-c']-dithiophen-4,8-dione.